(19) Europäisches Patentamt
European Patent Office
Office européen des brevets

(11) **EP 2 046 707 B1**

(12) **FASCICULE DE BREVET EUROPEEN**

(45) Date de publication et mention
de la délivrance du brevet:
**26.04.2017 Bulletin 2017/17**

(21) Numéro de dépôt: **07823625.4**

(22) Date de dépôt: **23.07.2007**

(51) Int Cl.:
*C07C 31/26* (2006.01)    *A23L 29/30* (2016.01)
*A23L 27/30* (2016.01)    *A23P 10/20* (2016.01)
*A61K 9/16* (2006.01)    *A61K 9/20* (2006.01)

(86) Numéro de dépôt international:
**PCT/FR2007/051707**

(87) Numéro de publication internationale:
**WO 2008/012465 (31.01.2008 Gazette 2008/05)**

(54) **SORBITOL GRANULE ET SON PROCEDE DE PREPARATION**

GRANULATFÖRMIGES SORBITOL UND VERFAHREN ZU SEINER HERSTELLUNG

GRANULATED SORBITOL AND METHOD FOR THE PREPARATION THEREOF

(84) Etats contractants désignés:
**AT BE BG CH CY CZ DE DK EE ES FI FR GB GR
HU IE IS IT LI LT LU LV MC MT NL PL PT RO SE
SI SK TR**

(30) Priorité: **28.07.2006 FR 0606954**

(43) Date de publication de la demande:
**15.04.2009 Bulletin 2009/16**

(73) Titulaire: **Roquette Frères
62136 Lestrem (FR)**

(72) Inventeurs:
• **DUFLOT, Pierrick
F-62136 La Couture (FR)**
• **BOIT, Baptiste
F-62400 Bethune (FR)**
• **LEFEVRE, Philippe
F-59660 Haverskerque (FR)**
• **LIS, José
F-59253 La Gorgue (FR)**

(74) Mandataire: **Cabinet Plasseraud
66 rue de la Chaussée d'Antin
75440 Paris Cedex 09 (FR)**

(56) Documents cités:
**EP-A1- 1 008 602    GB-A- 2 046 743**

**Description**

La présente invention a pour objet un sorbitol granulé de forme cristalline essentiellement $\gamma$ et à haute teneur en sorbitol, de surface spécifique, de comprimabilité et de granulométrie particulières.

Au sens de l'invention, on entend par « de forme cristalline essentiellement $\gamma$ », une teneur en cristaux de sorbitol de forme $\gamma$ supérieure à 95 % en poids, de préférence supérieure à 98 % en poids, plus préférentiellement encore supérieure à 99 % en poids.

La détermination de la nature des formes cristallines du sorbitol granulé conforme à l'invention pourra être réalisée par analyse microcalorimétrique ou DSC (Differential Scanning Calorimetry), technique qui permet principalement de déterminer la chaleur de fusion (enthalpie de fusion ou $\Delta Hf$) et la température de fusion (Tf) de polymorphes des produits soumis à l'analyse. Les 5 formes cristallines du sorbitol, i.e. les formes $\alpha$, $\beta$, $\gamma$, $\delta$ et $\varepsilon$ sont caractérisées par leur température de fusion respective. L'analyse est effectuée sur 6 mg d'échantillon de produit à analyser, finement broyé au mortier de laboratoire, déposé sans un creuset en aluminium d'une capacité de 40 $\mu$l, serti et percé d'un trou (appareil de mesure METTLER, de type DSC 821e). Le programme de montée en température consiste à passer de 10°C à 110°C à la vitesse de 2°C par minute. Les formes cristallines du sorbitol granulé conforme à l'invention sont identifiées sur le thermogramme DSC dans la plage de température de fusion comprise entre 98 et 99,5°C pour la forme cristalline $\gamma$. Pour l'intégration des pics de fusion correspondant à la forme $\gamma$, il est procédé ainsi : intégration du pic de fusion en utilisant la ligne de base à la droite du pic. La détermination est effectuée via un logiciel de mesure standard, par calcul de la chaleur (enthalpie) de fusion ($\Delta.Hf$ en J/g) et de la température de fusion (Tf) au sommet dudit pic,

La détermination du pourcentage en poids de la fraction cristalline de forme $\gamma$ du sorbitol granulé est réalisée par le rapport des enthalpies de fusion de chacune des formes cristallines ramenées à la somme des enthalpies de fusion mesurées pour un échantillon donné.

Au sens de l'invention, une « haute teneur en sorbitol » s'entend d'une teneur en sorbitol supérieure à 98 %, de préférence supérieure à 98,5 % en poids, plus préférentiellement encore comprise entre 98,5 et 99,5 % en poids sur sec.

La présente invention est également relative à un sorbitol granulé présentant une hygroscopicité, une masse volumique et une aptitude à l'écoulement tout à fait particulières.

L'invention concerne enfin un sorbitol granulé dont les propriétés techniques d'utilisation comme support d'arômes et en compression directe sont améliorées, ainsi qu'un procédé pour sa préparation.

Le sorbitol est un hexitol principalement utilisé dans les domaines d'applications alimentaire et pharmaceutique à titre d'agent sucrant, mais également pour sa caloricité réduite et son acariogénicité.

Le sorbitol, tout comme les autres polyols tels le xylitol ou le mannitol, est d'usage courant comme excipient pharmaceutique, comme édulcorant et agent de texture en industrie alimentaire, et comme support d'additif dans tous types d'industries. Il est cependant meilleur excipient que le xylitol et le mannitol, notamment en compression, en raison de son aptitude particulière à cristalliser sous forme de cristaux en aiguilles, directement compressibles.

En général, pour disposer d'un sorbitol cristallisé de grande résistance à la compression, on s'efforce de fabriquer un sorbitol de forme cristalline $\gamma$ (les formes $\alpha$ et $\beta$ sont particulièrement instables) en travaillant une solution sursaturée en sorbitol dont la forme $\gamma$ représente au moins 90 %.

Cependant, même lorsqu'il est cristallisé sous cette forme $\gamma$ la plus stable, le sorbitol granulé obtenu classiquement présente un certain nombre d'inconvénients dont celui d'être très hygroscopique.

Cette hygroscopicité élevée conduit à rendre l'écoulement du sorbitol granulé difficile, voire impossible, dès lors qu'une reprise en eau est intervenue.

Pour éviter ce problème d'écoulement du sorbitol granulé, il a été préconisé dans le brevet FR 1.506.334 de préparer un sorbitol de densité faible et de granulométrie plus grossière (comprise entre 0,42 et 1,19 mm).

Cependant, il est établi que plus la densité apparente d'un sorbitol granulé est faible, plus celui-ci devient friable, c'est-à-dire sensible à une altération de sa granulométrie par action mécanique. En outre, les temps de dissolution de ce produit granulé de granulométrie grossière sont en général trop longs et donc inadaptés.

Enfin, si l'aptitude à l'écoulement est partiellement améliorée par la mise en oeuvre de particules d'une telle granulométrie, le caractère hygroscopique résiduel encore trop élevé rend dans tous les cas l'utilisation de ce sorbitol granulé rédhibitoire lorsqu'il est associé à des ingrédients ou à des additifs très sensibles à l'eau.

Il est également établi que la capacité à fixer des quantités importantes d'additif est directement fonction de la surface spécifique desdites particules.

Les capacités d'absorption du sorbitol granulé sont ainsi d'autant plus importantes que sa surface spécifique est élevée. Cependant, il est connu que la surface spécifique des cristaux denses de $\gamma$ sorbitol du commerce est très faible.

Ainsi, pour une taille de particules comprise entre 500 et 1000 $\mu$m, elle est au plus égale à 0,7 m$^2$/g.

Dans le but de préparer un sorbitol sec présentant une meilleure granulométrie, une bonne aptitude à l'écoulement et satisfaisant aux conditions de compressibilité souhaitées, la demande de brevet FR 2.622.190 décrit un sorbitol poudre renfermant des particules d'un diamètre moyen relativement élevé compris entre 350 et 500 $\mu$m.

Cependant, la densité apparente élevée et la surface spécifique faible, de l'ordre de 0,9 à 1,2 m$^2$/g, ne sont pas significativement modifiées par le procédé de fabrication par atomisation mis en oeuvre, de sorte que le sorbitol ainsi obtenu conserve le même facteur d'absorption d'humidité et la même solubilité dans l'eau que la poudre de sorbitol de départ.

Le brevet EP 32.288 décrit un polymorphe de sorbitol de structure cristalline désintégrée et lâche, présentant une hygroscopicité améliorée et des propriétés de compression satisfaisantes. Cependant, ces propriétés particulières ne sont relatives qu'à une coupe granulométrique comprise entre 250 et 841 μm (i.e. 60/20 mesh), dont la surface spécifique est inférieure, en tout état de cause, à 2 m$^2$/g.

Le brevet EP 380.219 décrit des formes polymorphes de sorbitol à écoulement libre, présentant une surface spécifique pouvant atteindre 5 m$^2$/g, dont la masse volumique apparente est élevée (jusqu'à 0,7 g/ml) et la vitesse de solubilité dans l'eau satisfaisante.

Cependant, pour parvenir à ce résultat, il est nécessaire de préparer par atomisation des sphérules à centre creux de microcristaux aciculaires d'épaisseur inférieur à 1 μm et de longueur comprise entre 5 et 20 μm.

Par ailleurs, il est recommandé dans ce brevet de réaliser des mélanges sorbitol / mannitol ou d'ajouter du saccharose.

Le document GB 2 046 6 743 A décrit la fabrication du sorbitol comprimable comprenant au moins 90% en poids de sorbitol sous forme γ dont la surface comprend des micro-aiguilles orientées.

De tout ce qui précède, il ressort qu'il existe un besoin non satisfait de disposer d'un sorbitol granulé qui présente une surface spécifique et une comprimabilité les plus élevées possible, tout en conservant une granulométrie, une masse volumique et un écoulement libre satisfaisants.

La société Demanderesse a donc eu le mérite de concilier tous ces objectifs réputés jusqu'alors inconciliables en imaginant et élaborant, au prix de nombreuses recherches, un nouveau sorbitol granulé.

Le sorbitol granulé conforme à l'invention est ainsi tout d'abord caractérisé en ce qu'il présente :

- une surface spécifique, déterminée selon la méthode BET, supérieure ou égale à 2 m$^2$/g, de préférence comprise entre 2,2 et 4 m$^2$/g et plus préférentiellement encore comprise entre 2,5 et 3,5 m$^2$/g,
- une comprimabilité comprise entre 200 et 400 N, de préférence compris entre 250 et 350 N, et
- un diamètre moyen volumique mesuré par granulométrie à diffraction LASER à l'aide d'un module voie sèche compris entre 260 et 500 μm, de préférence compris entre 260 et 350 μm.

On détermine la surface spécifique sur la coupe granulométrique 250 - 841 μm du sorbitol granulé grâce à un analyseur de surface spécifique de marque BECKMAN-COULTER, de type SA3100 basé sur un test d'absorption de l'azote sur la surface du produit soumis à l'analyse, en suivant la technique décrite dans l'article BET Surface Area by Nitrogen Absorption de S. BRUNAUER et al. (Journal of American Chemical Society, 60, 309, 1938).

L'analyse BET est réalisée en 3 points.

Le sorbitol granulé conforme à l'invention présente alors une surface spécifique supérieure ou égale à 2 m$^2$/g, de préférence comprise entre 2,2 et 4 m$^2$/g et plus préférentiellement encore comprise entre 2,5 et 3,5 m$^2$/g, ce qui est remarquablement élevé.

La comprimabilité du sorbitol granulé est déterminée de la façon suivante.

On prépare des comprimés de sorbitol à l'aide d'une presse alternative de laboratoire FROGERAIS AM équipée de poinçons ronds concaves de diamètre 13 mm et de rayon de courbure de 13 mm. Le lubrifiant utilisé est le stéarate de magnésium au taux de 1 %.

Les comprimés fabriqués sont d'épaisseur constante (5 mm) et de poids variable, d'où des densités de comprimés variables qui permettent de tracer les graphes dureté en fonction de la densité des comprimés. La dureté est mesurée sur un duromètre ERWEKA TBH30GMD, et est exprimée en Newtons. La comprimabilité du sorbitol granulé conforme à l'invention, mesurée pour des comprimés de densité 1,3 g/ml, est déterminée à une valeur comprise entre 200 et 400 N, de préférence compris entre 250 et 350 N.

Grâce à cette valeur de comprimabilité remarquable, la résistance mécanique des comprimés obtenus avec ledit sorbitol granulé conforme à l'invention est très élevée, comparativement à celle des comprimés obtenus avec les produits du commerce.

A titre d'exemples parmi les sorbitol accessibles dans le commerce, la société Demanderesse a trouvé que ceux présentant la surface spécifique la plus élevée sont notamment ceux commercialisés par la société MERCK sous l'appellation KARION®, tel le KARION® P 300 qui présente une surface spécifique selon BET pouvant atteindre 3,4 m$^2$/g.

La comprimabilité de cet échantillon, déterminée dans les mêmes conditions que celles utilisées pour caractériser le sorbitol granulé conforme à l'invention, ne dépasse pas une valeur de l'ordre de 320 N.

Quant au sorbitol pulvérulent fabriqué par la société Demanderesse selon l'enseignement de son brevet EP 1.008.602, s'il présente une comprimabilité, mesurée pour des comprimés de densité 1,3 g/ml, de l'ordre de 275 N, c'est pour un sorbitol pulvérulent d'un diamètre moyen de particules inférieur à 200 μm et une surface spécifique de 2,4 m$^2$/g.

Le sorbitol granulé conforme à l'invention est également caractérisé par son diamètre moyen volumique (moyenne arithmétique) D4,3, compris entre 260 et 1000 μm, de préférence compris entre 260 et 500 μm, et plus préférentiellement encore compris entre 260 et 350 μm.

Ces valeurs sont déterminées sur un granulomètre à diffraction LASER type LS 230 de la société BECKMAN-COULTER, équipé de son module de dispersion poudre (voie sèche), en suivant le manuel technique et les spécifications du constructeur.

La gamme de mesure du granulomètre à diffraction LASER type LS 230 est de 0,04 μm à 2.000 μm.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

Les résultats sont calculés en % volumique, et exprimés en μm.

A la connaissance de la société Demanderesse, il n'existe pas de sorbitol granulé, dans cette coupe granulométrique de 260 à 500 μm, qui présente de telles valeurs de surface spécifique et de compression.

Une fois encore, à titre d'exemple, l'échantillon de sorbitol KARION P300 de MERCK analysé par la société Demanderesse présente un diamètre moyen compris entre 200 et 250 μm, et le sorbitol pulvérulent fabriqué par la société Demanderesse selon l'enseignement de son brevet EP 1.008.602, présente quant à lui un diamètre moyen compris entre 150 et 250 μm.

Le sorbitol granulé de l'invention présente une hygroscopicité, déterminée par son évolution pondérale entre 60 et 0 % d'humidité relative (HR), comprise entre 1 et 2 %.

Le test de mesure de l'hygroscopicité consiste ici à évaluer la variation de poids de l'échantillon de sorbitol lorsqu'il est soumis à différentes HR à 20°C dans un équipement fabriqué par la société SURFACE MEASUREMENT SYSTEMS (Londres UK) et dénommé Dynamic Vapour Sorption Série 1.

Cet équipement consiste en une microbalance différentielle qui permet de quantifier l'évolution pondérale d'un échantillon par rapport à une référence (ici la nacelle de référence de la balance différentielle est vide) lorsque celui-ci est soumis à différentes conditions climatiques.

Le gaz vecteur est l'azote, et le poids de l'échantillon est compris entre 10 et 12 mg. Les HR programmées sont 0 % HR pendant 24 h (déshydratation) puis 10, 20, 30, 35, 40, 45, 50, 52, 54,56, 58 et 60 % HR. Le facteur de stabilité qui permet le passage automatique d'une HR à la suivante est le rapport dm/dt qui est fixé à 0,002 %/mn pendant 20 minutes.

En final, est obtenue une table de valeurs correspondant pour chaque HR à l'équation $[(m-m_0)/m_0] \times 100$ où « m » est la masse de l'échantillon en fin de test pour l'HR considérée et « $m_0$ » la masse en fin de déshydratation.

Les résultats sont exprimés comme la différence entre les valeurs d'évolution pondérale (telles que décrites ci-dessus) obtenues respectivement à 60 % et après déshydratation (à 0 % HR).

Il est particulièrement surprenant qu'un sorbitol granulé puisse présenter conjointement une surface spécifique supérieure ou égale à 2 m$^2$/g, de préférence comprise entre 2,2 et 4 m$^2$/g, plus préférentiellement encore comprise entre 2,5 et 3,5 m$^2$/g, et une hygroscopicité aussi basse, i.e. comprise entre 1 et 2 %.

En effet, il est admis très classiquement que l'hygroscopicité d'un produit augmente avec sa surface spécifique, i.e. sa surface exposée au milieu contenant de la vapeur d'eau.

Or, le sorbitol granulé conforme à l'invention présente une surface spécifique élevée, caractéristique d'un produit granulé, avec pourtant une faible hygroscopicité, caractéristique d'un produit cristallisé sous une forme cristalline stable.

A titre d'exemples, l'échantillon de sorbitol KARION® P 300 commercialisé par la société MERCK et analysé par la société Demanderesse présente une hygroscopicité de 2,4 % pour une surface spécifique selon BET de 3,4 m$^2$/g.

Le sorbitol granulé conforme à l'invention est également caractérisé par sa masse volumique et son écoulement libre.

La masse volumique du sorbitol granulé conforme à l'invention est réalisée par la détermination du rapport entre la masse de l'échantillon à analyser et le volume qu'il occupe après un écoulement libre dans un récipient et à une température donnée.

Plus précisément, il est procédé à la mesure à température ambiante, d'un volume d'échantillon contenu dans une éprouvette de 250 ml et la détermination dudit volume par mesure de la masse d'un égal volume d'eau à température ambiante.

Après lavage et séchage de l'éprouvette, on la pèse avec une précision de 0,1 g (détermination de la masse initiale ou $m_o$), la remplie à ras bord d'eau et la pèse à nouveau (soit masse m).

On vide ladite éprouvette, la lave et la sèche à nouveau, et introduit l'échantillon dans l'éprouvette à l'aide d'une trémie en inox en forme d'entonnoir (diamètre supérieur : 12 cm ; diamètre intérieur : 12 mm ; hauteur du cône : 9 cm ; longueur de la tubulure : 2 cm) disposée sur un support de manière à ce que la hauteur comprise entre la trémie et l'éprouvette soit de 10 cm.

On laisse ensuite l'échantillon s'écouler librement par la trémie dans l'éprouvette, jusqu'à ce que celle-ci soit remplie à ras bord.

On arase le surplus du produit à l'aide d'une spatule, de façon à obtenir une surface plane au sommet de l'éprouvette.

On enlève l'éprouvette, et la pèse avec son contenu, toujours avec une précision de 0,1 g (soit masse $m_2$).

La masse volumique, exprimée en g/l, est donnée par la formule suivante :

$$[(m_2 - m_0) / (m_1 - m_c)] \times \rho \times 1000$$

où $\rho$ est la masse volumique, en g/ml, de l'eau à la température ambiante.

Dans ces conditions, le sorbitol granulé conforme à l'invention présente une masse volumique comprise entre 350 et 650 g/l, de préférence comprise entre 400 et 550 g/l.

Quant à l'écoulement libre du sorbitol granulé conforme à l'invention, il est déterminé selon la méthode de mesure préconisée par la Pharmacopée Européenne (PE 5.0 tome 1, 01/2005 : 20916 article 2.9.16).

Le sorbitol granulé de l'invention présente alors un écoulement libre compris entre 5 et 15 secondes.

Cette valeur est tout à fait satisfaisante, en regard de celles des poudres de sorbitol de l'art antérieur.

L'ensemble de ces propriétés technologiques rend le sorbitol granulé conforme à l'invention particulièrement apte à être utilisé en tant qu'agent édulcorant, agent de texture, excipient et surtout comme support d'additif dans des compositions sous forme de tablettes ou de comprimés destinés en particulier aux domaines alimentaires, pharmaceutiques et industriels.

Selon un premier mode de réalisation, le sorbitol granulé conforme à l'invention est susceptible d'être obtenu en procédant à une étape de pulvérisation d'un fondu de sorbitol sur un sorbitol poudre dans un granulateur à lit d'air fluidisé.

Selon un second mode de réalisation, le sorbitol granulé conforme à l'invention est susceptible d'être obtenu en procédant à une étape de pulvérisation d'une solution de sorbitol à haute matière sèche sur un sorbitol poudre dans un granulateur à lit d'air fluidisé.

Pour obtenir un sorbitol granulé conforme à l'invention possédant les caractéristiques fonctionnelles énoncées, la société Demanderesse a constaté qu'il convenait de choisir comme sorbitol de départ un sorbitol poudre, pouvant être obtenu par cristallisation dans l'eau ou dans un autre solvant comme l'alcool, par dragéification, par atomisation ou par extrusion.

Dans le premier mode de réalisation, le fondu est constitué de sorbitol ayant une matière sèche supérieure à 98,5 %, de préférence supérieure à 99 % obtenu par évaporation sous vide d'une solution de sorbitol.

Dans le second mode de réalisation, la solution de sorbitol à haute matière sèche présente quant à elle une matière sèche comprise entre 65 et 98,5 %, de préférence de comprise entre 70 % et 98,5 %, plus préférentiellement encore comprise entre 90 et 98,5 %.

De manière surprenante et inattendue, la société Demanderesse a constaté que la granulation d'un sorbitol à l'aide d'un fondu, ou à l'aide d'une solution de sorbitol à haute matière sèche, dans un granulateur à lit d'air fluidisé permet de préparer avec un haut rendement un produit conforme à l'invention sur le plan de sa surface spécifique et de sa comprimabilité, de sa granulométrie et son hygroscopicité, de sa masse volumique et sa vitesse d'écoulement.

En effet, les procédés décrits antérieurement ne permettent pas d'obtenir l'ensemble des caractéristiques souhaitées.

Pour procéder à la granulation, on peut employer par exemple un granulateur à lit d'air fluidisé continu.

Il peut être choisi avantageusement un granulateur à lit d'air fluidisé continu circulaire avec tube de déchargement, ou continu rectangulaire à écoulement piston, dans lequel on introduit, via un doseur pondéral, le sorbitol poudre en continu et via un doseur volumétrique, le fondu de sorbitol, ou la solution de sorbitol à haute matière sèche, à pulvériser en continu.

Comme il sera exemplifié ci-après, la société Demanderesse a choisi d'employer un granulateur à lit d'air fluidisé continu avec tube classificateur de type AGT commercialisé par la société GLATT.

La bonne mise en contact des constituants et la granulation du fondu, ou de la solution de sorbitol à haute matière sèche, sur les particules de la poudre de sorbitol sont réalisées par mise en suspension dans un flux d'air.

C'est ainsi que la poudre de sorbitol et le fondu de sorbitol, ou la solution de sorbitol à haute matière sèche, sont granulés par mise en contact dans le lit d'air fluidisé équipé d'un système de pulvérisation de liquides par buses d'injection, par exemple bi-fluides.

A la sortie du granulateur à lit d'air fluidisé, les granulés sont déchargés en continu. Le déchargement se fait préférentiellement par le tube classificateur du granulateur AGT, et par débordement, si le granulateur continu rectangulaire à écoulement piston est utilisé.

Le sorbitol granulé conforme à l'invention est obtenu après refroidissement et éventuellement tamisage. Dans ce cas, les fines particules, encore appelés les fines, peuvent être directement recyclées en tête de granulation et les grosses particules être broyées pour donner ce qui est appelé les refus broyés puis recyclées en tête de tamisage ou en tête de granulation.

Une fraction du sorbitol granulé conforme à l'invention peut également être broyé et recyclé en tête de granulation.

Dans un mode préférentiel de réalisation du procédé conforme à l'invention, on effectue les étapes successives suivantes :

a) éventuellement concentrer par évaporation une solution présentant une matière sèche en sorbitol comprise entre 65 et 75 %, plus préférentiellement de 70 %, de manière à obtenir un fondu ayant une teneur en matière sèche supérieure ou égale à 99 %,

b) introduire, dans un granulateur à lit d'air fluidisé muni d'un tube de déchargement, ledit fondu de sorbitol avec un sorbitol cristallisé dans un ratio en poids sorbitol cristallisé : fondu de sorbitol ou solution de sorbitol à haute matière sèche, supérieur à 0,5 : 1, de préférence compris entre de 1 : 1 et 2 : 1,

c) granuler le sorbitol cristallisé avec ledit fondu, à une température de lit d'air fluidisé supérieure à 65°C, de préférence comprise entre 75°C et 85°C,

d) récupérer le produit granulé ainsi obtenu via le tube de déchargement,

e) refroidir le ledit produit granulé ainsi obtenu à température ambiante, typiquement 20°C, pendant une durée comprise entre 30 minutes et 2 heures, de préférence égale à 1 heure,

f) tamiser et récupérer le sorbitol granulé ainsi obtenu.

De manière avantageuse le procédé pourra comprendre l'étape ultérieure suivante :

g) recycler les fines, les refus broyés et une fraction du sorbitol granulé ainsi obtenu de manière à maintenir un ratio en poids poudre de sorbitol : fondu de sorbitol ou solution de sorbitol à haute matière sèche supérieur à 0,5 : 1, de préférence compris entre 1 : 1 et 2 : 1.

L'invention concerne également l'utilisation de sorbitol granulé conforme à l'invention en tant qu'agent édulcorant, agent de texture, excipient ou surtout comme support d'additif dans des compositions sous forme de comprimés destinés en particulier aux domaines alimentaires, pharmaceutiques ou industriels.

L'invention concerne également un comprimé fabriqué à partir de sorbitol granulé conforme à l'invention. La teneur en sorbitol du comprimé dépendra de l'usage souhaité du comprimé. Typiquement la teneur en sorbitol du comprimé pourra être comprise entre 1% et 90%.

D'autres caractéristiques et avantages de l'invention apparaîtront à la lecture des exemples qui suivent. Ils ne sont toutefois donnés ici qu'à titre illustratif et non limitatif.

## Exemple 1

Une solution ayant une teneur de 70 % en sorbitol sur la base de la matière sèche est placée dans un récipient d'évaporation sous vide pour obtenir un fondu de sorbitol ayant une teneur en matière sèche de 99 %.

En fin d'évaporation, le fondu est maintenu à une température de 120 °C.

On alimente alors un granulateur à lit d'air fluidisé de type AGT 400, préalablement chargé avec 25 kg de sorbitol cristallisé, en continu via un doseur poudre commercialisé par la société KTRON, à un débit d'environ 10 kg/h, avec un sorbitol cristallisé commercialisé par la société Demanderesse sous le nom de marque NEOSORB®, de manière à obtenir un diamètre moyen volumique de particules de 180 $\mu$m.

D'autre part, on alimente le granulateur à lit d'air fluidisé en continu, via une buse de pulvérisation bi-fluide à un débit de 10 kg/h avec le fondu de sorbitol.

La pulvérisation du fondu est assurée par un air à 120°C sous une pression de 5,5 bars.

L'air utilisé pour la mise en suspension du sorbitol granulé a un débit d'environ 850 m$^3$/h et une température réglée de manière à ce que la température dans le lit d'air fluidisé soit de 80°C

Le sorbitol granulé est déchargé en continu via le tube classificateur (le débit d'air dans le tube est réglé à 60 m$^3$/h de manière à obtenir la bonne granulométrie finale) dans un lit d'air fluidisé afin d'être refroidit de 80°C à 20°C en une heure.

Le produit granulé et refroidi est ensuite tamisé en continu sur un tamis rotatif équipé de deux toiles métalliques de 100 et 500 $\mu$m.

Les fines, les refus broyés et une fraction du produit obtenu entre 100 et 500 $\mu$m, lui aussi broyé (broyage effectué de manière à obtenir une taille de particules moyenne du recyclage de l'ordre de 180 $\mu$m) sont recyclés en tête de granulation de manière à maintenir un débit total de recyclage de 10 kg/h.

Le sorbitol granulé ainsi obtenu présente les caractéristiques rassemblées dans le tableau I suivant.

Tableau I

| Paramètres | Sorbitol granulé de l'invention |
|---|---|
| Analyses DSC :<br>Forme $\gamma$<br>    $\triangle$Hf (J/g)<br>    Tf | <br><br>173<br>99,2 |
| Autres formes<br>    $\triangle$Hf (J/g) | <br>nd |
| Surface spécifique selon BET ($m^2$/g) | 2,4 |
| Comprimabilité (N) (dureté ERWEKA des comprimés) | 270 |
| Diamètre moyen LASER ($\mu$m) | 338 |
| Hygroscopicité | 1,10 |
| Masse volumique (g/l) | 483 |
| Ecoulement libre (s) | 12 |

**Exemple 2**

Une solution ayant une teneur de 70 % en sorbitol sur la base de la matière sèche est placée dans un récipient d'évaporation sous vide pour obtenir un fondu de sorbitol ayant une teneur en matière sèche de 99 %.

En fin d'évaporation, le fondu est maintenu à une température de 120 °C.

On alimente alors un granulateur à lit d'air fluidisé de type AGT 400, préalablement chargé avec 25 kg de sorbitol cristallisé, en continu via un doseur poudre commercialisé par la société KTRON, à un débit d'environ 20 kg/h, avec un sorbitol cristallisé commercialisé par la société Demanderesse sous le nom de marque NEOSORB®, de manière à obtenir un diamètre moyen de particules de 180 $\mu$m.

D'autre part, on alimente le granulateur à lit d'air fluidisé en continu, via une buse de pulvérisation bi-fluide à un débit de 10 kg/h avec le fondu de sorbitol.

La pulvérisation du fondu est assurée par un air à 120°C sous une pression de 5,5 bars.

L'air utilisé pour la mise en suspension du sorbitol granulé a un débit d'environ 850 $m^3$/h et une température réglée de manière à ce que la température dans le lit d'air fluidisé soit de 80°C.

Le sorbitol granulé est déchargé en continu via le tube classificateur (le débit d'air est réglé à 60 $m^3$/h de manière à obtenir la bonne granulométrie finale) dans un lit d'air fluidisé afin d'être refroidit de 80°C à 20°C en une heure.

Le produit granulé et refroidi est ensuite tamisé en continu sur un tamis rotatif équipé de deux toiles métalliques de 100 et 500 $\mu$m.

Les fines, les refus broyés et une fraction du produit obtenu entre 100 et 500 $\mu$m, lui aussi broyé (broyage effectué de manière à obtenir une taille de particules moyenne du recyclage de l'ordre de 180 $\mu$m) sont recyclés en tête de granulation de manière à maintenir un débit total de recyclage de 20 kg/h.

Le sorbitol granulé ainsi obtenu présente les caractéristiques rassemblées dans le tableau II suivant.

Tableau II

| Paramètres | Sorbitol granulé de l'invention |
|---|---|
| Analyses DSC :<br>Forme $\gamma$<br>    $\triangle$Hf (J/g)<br>    Tf | <br><br>179<br>99,2 |
| Autres formes<br>    $\triangle$Hf (J/g) | <br>nd |
| Surface spécifique selon BET ($m^2$/g) | 2,9 |
| Comprimabilité (N) (dureté ERWEKA des comprimés) | 275 |
| Diamètre moyen LASER ($\mu$m) | 270 |

(suite)

| Paramètres | Sorbitol granulé de l'invention |
|---|---|
| Hygroscopicité | 1,11 |
| Masse volumique (g/l) | 523 |
| Ecoulement libre (s) | 7 |

**Exemple 3**

On alimente un granulateur à lit d'air fluidisé de type AGT 400, préalablement chargé avec 25 kg de sorbitol cristallisé, en continu via un doseur poudre commercialisé par la société KTRON, à un débit d'environ 12 kg/h, avec un sorbitol cristallisé commercialisé par la société Demanderesse sous le nom de marque NEOSORB®, de manière à obtenir un diamètre moyen de particules de 180 $\mu$m.

D'autre part, on alimente le granulateur à lit d'air fluidisé en continu, via une buse de pulvérisation bi-fluide à un débit de 18 kg/h avec une solution de sorbitol à 70% de matière sèche ayant une température de 60°C.

La pulvérisation de la solution est assurée par un air à 90°C sous une pression de 3 bars.

L'air utilisé pour la mise en suspension du sorbitol granulé a un débit d'environ 850 m³/h et une température réglée de manière à ce que la température dans le lit d'air fluidisé soit de 75°C.

Le sorbitol granulé est déchargé en continu via le tube classificateur (le débit d'air dans le tube est réglé à 60 m³/h de manière à obtenir la bonne granulométrie finale) dans un lit d'air fluidisé afin d'être refroidit de 75°C à 20°C en une heure.

Le produit granulé et refroidi est ensuite tamisé en continu sur un tamis rotatif équipé de deux toiles métalliques de 100 et 500 $\mu$m.

Les fines, les refus broyés et une fraction du produit obtenu entre 100 et 500 $\mu$m, lui aussi broyé (broyage effectué de manière à obtenir une taille de particules moyenne du recyclage de l'ordre de 180 $\mu$m) sont recyclés en tête de granulation de manière à maintenir un débit total de recyclage de 12 kg/h.

Le sorbitol granulé ainsi obtenu présente les caractéristiques rassemblées dans le tableau III suivant.

Tableau III

| Paramètres | Sorbitol granulé de l'invention |
|---|---|
| Analyses DSC : Forme $\gamma$ | |
| $\Delta$Hf (J/g) | 165 |
| Tf | 98,7 |
| Autres formes | |
| $\Delta$Hf (J/g) | 2 |
| Surface spécifique selon BET (m²/g) | 2,6 |
| Comprimabilité (N) (dureté ERWEKA des comprimés) | 340 |
| Diamètre moyen LASER ($\mu$m) | 328 |
| Hygroscopicité | 1,85 |
| Masse volumique (g/l) | 470 |
| Ecoulement libre (s) | 11 |

**Exemple 4**

Une solution ayant une teneur de 70 % en sorbitol sur la base de la matière sèche est placée dans un récipient d'évaporation pour obtenir une solution de sorbitol ayant une teneur en matière sèche de 90 %.

En fin d'évaporation, la solution est maintenue à une température de 110°C.

On alimente alors un granulateur à lit d'air fluidisé de type AGT 400, préalablement chargé avec 25 kg de sorbitol cristallisé, en continu via un doseur poudre commercialisé par la société KTRON, à un débit d'environ 15 kg/h, avec un sorbitol cristallisé commercialisé par la société Demanderesse sous le nom de marque NEOSORB®, de manière à obtenir un diamètre moyen de particules de 180 μm.

D'autre part, on alimente le granulateur à lit d'air fluidisé en continu, via une buse de pulvérisation bi-fluide à un débit de 15 kg/h avec la solution de sorbitol.

La pulvérisation du fondu est assurée par un air à 110°C sous une pression de 4,5 bars.

L'air utilisé pour la mise en suspension du sorbitol granulé a un débit d'environ 800 m$^3$/h et une température réglée de manière à ce que la température dans le lit d'air fluidisé soit de 80°C

Le sorbitol granulé est déchargé en continu via le tube classificateur (le débit d'air dans le tube est réglé à 60 m $^3$/h de manière à obtenir la bonne granulométrie finale) dans un lit d'air fluidisé afin d'être refroidit de 80°C à 20°C en une heure.

Le produit granulé et refroidi est ensuite tamisé en continu sur un tamis rotatif équipé de deux toiles métalliques de 100 et 500 μm.

Les fines, les refus broyés et une fraction du produit obtenu entre 100 et 500 μm, lui aussi broyé (broyage effectué de manière à obtenir une taille de particules moyenne du recyclage de l'ordre de 180 μm) sont recyclés en tête de granulation de manière à maintenir un débit total de recyclage de 15 kg/h.

Le sorbitol granulé ainsi obtenu présente les caractéristiques rassemblées dans le tableau IV suivant.

Tableau IV

| Paramètres | Sorbitol granulé de l'invention |
|---|---|
| Analyses DSC : <br> Forme γ <br> ΔHf (J/g) <br> Tf | <br><br> 167 <br> 98,9 |
| Autres formes <br> ΔHf (J/g) | <br> <1 |
| Surface spécifique selon BET (m$^2$/g) | 2,3 |
| Comprimabilité (N) (dureté ERWEKA des comprimés) | 280 |
| Diamètre moyen LASER (μm) | 303 |
| Hygroscopicité | 1,63 |
| Masse volumique (g/l) | 505 |
| Ecoulement libre (s) | 10 |

**Exemple 5**

On alimente un granulateur à lit d'air fluidisé de type AGT 400, préalablement chargé avec 25 kg de sorbitol cristallisé, en continu via un doseur poudre commercialisé par la société KTRON, à un débit d'environ 13 kg/h, avec un sorbitol cristallisé commercialisé par la société Demanderesse sous le nom de marque NEOSORB®, de manière à obtenir un diamètre moyen de particules de 450 μm.

D'autre part, on alimente le granulateur à lit d'air fluidisé en continu, via une buse de pulvérisation bi-fluide à un débit de 17 kg/h avec une solution de sorbitol à 70% de matière sèche ayant une température de 60°C.

La pulvérisation de la solution est assurée par un air à 90°C sous une pression de 2.5 bars.

L'air utilisé pour la mise en suspension du sorbitol granulé a un débit d'environ 900 m $^3$/h et une température réglée de manière à ce que la température dans le lit d'air fluidisé soit de 67°C.

Le sorbitol granulé est déchargé en continu via le tube classificateur (le débit d'air dans le tube est réglé à 130 m $^3$/h de manière à obtenir la bonne granulométrie finale) dans un lit d'air fluidisé afin d'être refroidit de 67°C à 20°C en une heure.

Le produit granulé et refroidi est ensuite tamisé en continu sur un tamis rotatif équipé de deux toiles métalliques de 400 et 1300 μm.

Les fines, les refus broyés et une fraction du produit obtenu entre 400 et 1300 μm, lui aussi broyé (broyage effectué de manière à obtenir une taille de particules moyenne du recyclage de l'ordre de 450 μm) sont recyclés en tête de granulation de manière à maintenir un débit total de recyclage de 13 kg/h.

Le sorbitol granulé ainsi obtenu présente les caractéristiques rassemblées dans le tableau V suivant.

Tableau V

| Paramètres | Sorbitol granulé de l'invention |
|---|---|
| Analyses DSC :<br>Forme $\gamma$<br>$\Delta$Hf (J/g)<br>Tf | <br><br>173<br>99,0 |
| Autres formes<br>$\Delta$Hf (J/g) | <br>- |
| Surface spécifique selon BET (m$^2$/g) | 3,3 |
| Comprimabilité (N) (dureté ERWEKA des comprimés) | 300 |
| Diamètre moyen LASER ($\mu$m) | 880 |
| Hygroscopicité | 1,20 |
| Masse volumique (g/l) | |
| Ecoulement libre (s) | |

## Exemple 6

Les sorbitols granulés des exemples 1 à 4 sont comparés, dans le tableau VI suivant, à des sorbitols granulés connus par ailleurs.

Tableau VI

| | Sorbitol granulé Exemple 1 | Sorbitol granulé Exemple 2 | Sorbitol granulé Exemple 3 | Sorbitol granulé Exemple 4 | Sorbitol granulé Exemple 5 | KARION P 300 MERCK | Sorbitol préparé selon EP 1.008.602 |
|---|---|---|---|---|---|---|---|
| Surface spécifique selon BET (m$^2$/g) | 2,4 | 2,9 | 2,6 | 2,3 | 3,3 | 3,4 | 2,4 |
| Comprimabilité (N) (dureté ERWEKA des comprimés) | 270 | 275 | 340 | 280 | 300 | 320 | 275 |
| Diamètre moyen LASER ($\mu$m) | 338 | 270 | 328 | 303 | 880 | 220 | 155 |
| Hygroscopicité | 1,10 | 1,11 | 1,85 | 1,63 | 1,20 | 2,40 | 1,3 |
| Masse volumique (g/l) | 483 | 523 | 470 | 505 | 410 | 473 | 500 |
| Ecoulement libre (s) | 12 | 7 | 11 | 10 | 18 | 7 | 6 |

Les sorbitols granulés conformes à l'invention possèdent tous, contrairement aux produits de l'art antérieur, d'excellentes propriétés fonctionnelles, les rendant aptes à être utilisés sans inconvénient comme excipients et supports d'additifs non hygroscopiques, en particulier dans les industries alimentaires, pharmaceutiques et industrielles (par exemple dans les applications tablettes détergentes).

## Exemple 7

On évalue la dureté de comprimés, fabriqués avec le sorbitol granulé conforme à l'invention de l'exemple 1, et contenant un arôme et un édulcorant du commerce.

La composition des comprimés, exprimée en % en poids du comprimé, est donnée par le tableau VII suivant :

Tableau VII

| | |
|---|---|
| Sorbitol Granulé de l'invention | 98,1 % |
| Arôme menthe liquide GIVAUDAN | 1 % |
| ASPARTAM | 0,2 % |
| Stéarate de magnésium | 0,7 % |

Les comprimés sont ronds à faces bi-convexes de diamètre 8 mm et d'épaisseur 4,5 mm. Les duretés des comprimés ainsi fabriqués sont mesurées pour deux poids de comprimés distincts, en maintenant l'épaisseur constante. Les résultats obtenus sont présentés dans le tableau VIII suivant.

Tableau VIII

| | Comprimé 1 | Comprimé 2 |
|---|---|---|
| Poids (mg) | 231 | 253 |
| Dureté ERWEKA TBH30GMD (N) | 183 | 327 |

Il apparaît qu'une augmentation de poids du comprimé d'à peine 9,5 % permet d'en augmenter la dureté considérablement (79 %), ce qui conduit à un gain remarquable en dureté sans qu'il ne soit nécessaire d'introduire beaucoup plus de matière.

Ce résultat constitue un gain non négligeable pour l'utilisateur potentiel.

**Revendications**

1. Sorbitol granulé présentant une teneur en cristaux de sorbitol de forme $\gamma$ supérieure à 95 % en poids, ledit sorbitol granulé comprenant une teneur en sorbitol supérieure à 98 %, **caractérisé en ce qu'**il présente :

   - une surface spécifique, déterminée selon la méthode BET, supérieure ou égale à 2 m$^2$/g, de préférence comprise entre 2,2 et 4 m$^2$/g et plus préférentiellement encore comprise entre 2, et 3,5 m$^2$/g,
   - une comprimabilité comprise entre 200 et 400 N, de préférence compris entre 250 et 350 N, et
   - un diamètre moyen volumique mesuré par granulométrie à diffraction LASER à l'aide d'un module voie sèche compris entre 260 et 500 $\mu$m, de préférence compris entre 260 et 350 $\mu$m.

2. Sorbitol selon la revendication 1, **caractérisé en ce qu'**il présente une valeur d'hygroscopicité, déterminée par son évolution pondérale entre 60 % et 0 % d'humidité relative, comprise entre 1 et 2 %.

3. Sorbitol selon l'une ou l'autre des revendications 1 et 2, **caractérisé en ce qu'**il présente une masse volumique comprise entre 350 et 650 g/l, de préférence comprise entre 400 et 550 g/l.

4. Sorbitol selon l'une quelconque des revendications 1 à 3, **caractérisé en ce qu'**il présente un écoulement libre compris entre 5 et 15 secondes.

5. Procédé de préparation d'un sorbitol granulé selon l'une quelconque des revendications 1 à 4, **caractérisé en ce qu'**il comprend une étape de pulvérisation d'un fondu de sorbitol sur un sorbitol cristallisé dans un granulateur à lit d'air fluidisé.

6. Procédé selon la revendication 5, **caractérisé en ce qu'**il consiste dans la succession des étapes suivantes :

   a) concentrer par évaporation une solution présentant une matière sèche en sorbitol comprise entre 65 et 75 %, de préférence de 70 %, de manière à obtenir un fondu ayant une teneur en matière sèche supérieure ou égale à 99 %,

b) introduire, dans un granulateur à lit d'air fluidisé muni d'un tube de déchargement, ledit fondu de sorbitol avec un sorbitol cristallisé dans un ratio sorbitol cristallisé : fondu de sorbitol supérieur à 0,5 : 1, de préférence compris entre plus de 1 : 1 et 2 : 1,

c) granuler le sorbitol cristallisé avec ledit fondu, à une température de lit d'air fluidisé supérieure à 65°C, de préférence comprise entre 75°C et 85°C,

d) récupérer le produit granulé ainsi obtenu via le tube de déchargement,

e) refroidir le ledit produit granulé ainsi obtenu à une température ambiante, pendant une durée comprise entre 30 minutes et 2 heures, de préférence égale à 1 heure,

f) tamiser et récupérer le sorbitol granulé ainsi obtenu.

7. Procédé selon la revendication 6, comprenant l'étape ultérieure suivante :

g) recycler les fines, les refus broyés et une fraction du sorbitol granulé ainsi obtenu de manière à maintenir un ratio en poids de poudre de sorbitol : fondu de sorbitol supérieur à 0,5 : 1, de préférence compris entre 1 : 1 et 2 : 1

8. Utilisation de sorbitol granulé selon l'une quelconque des revendications 1 à 4 en tant qu'agent édulcorant, agent de texture, excipient et surtout comme support d'additif dans des compositions sous forme de comprimés.

9. Comprimé fabriqué à partir du sorbitol granulé selon l'une quelconque des revendications 1 à 4.

**Patentansprüche**

1. Granuliertes Sorbitol, welches einen Gehalt an Sorbitolkristallen der Form $\gamma$ von mehr als 95 Gew.-% aufweist, wobei das granulierte Sorbitol einen Gehalt an Sorbitol oberhalb von 98 % umfasst, **dadurch gekennzeichnet, dass** es aufweist:

- eine spezifische Oberfläche bestimmt nach dem BET-Verfahren, oberhalb von oder gleich 2 m$^2$/g, vorzugsweise umfasst von 2,2 und 4 m$^2$/g und stärker bevorzugt noch umfasst von 2,5 und 3,5 m$^2$/g,
- eine Kompressibilität umfasst von 200 und 400 N, vorzugsweise umfasst von 250 und 350 N, und
- einen mittleren Volumendurchmesser, gemessen durch Granulometrie mit einem Diffraktionslaser mit Hilfe eines Trockenmoduls, umfasst von 260 und 500 $\mu$m, bevorzugt umfasst von 260 und 350 $\mu$m.

2. Sorbitol nach Anspruch 1, **dadurch gekennzeichnet, dass** es einen Hygroskopizitätswert, bestimmt durch seine Gewichtsveränderung zwischen 60 % und 0 % relative Feuchtigkeit, umfasst von 1 und 2 % aufweist.

3. Sorbitol nach einem der Ansprüche 1 und 2, **dadurch gekennzeichnet, dass** es eine Volumenmasse umfasst von 350 und 650 g/l aufweist, vorzugsweise umfasst von 400 und 550 g/l.

4. Sorbitol nach einem der Ansprüche 1 bis 3, **dadurch gekennzeichnet, dass** es eine freie Strömung umfasst von 5 und 15 Sekunden aufweist.

5. Verfahren zur Herstellung eines granulierten Sorbitols nach einem der Ansprüche 1 bis 4, **dadurch gekennzeichnet, dass** es einen Schritt zur Pulverisierung einer Sorbitolschmelze über einem kristallisierten Sorbitol in einem Luftwirbelbett-Granulator umfasst.

6. Verfahren nach Anspruch 5, **dadurch gekennzeichnet, dass** es aus der Folge der folgenden Schritte besteht:

a) Konzentrieren durch Verdampfen einer Lösung, welche eine Trockenmasse an Sorbitol umfasst von 65 und 75 % aufweist, vorzugsweise von 70 %, um eine Schmelze zu erhalten, welche einen Trockenmassegehalt oberhalb von oder gleich 99 % aufweist,

b) Einbringen in einen Luftwirbelbett-Granulator ausgestattet mit einem Entladerohr, der Sorbitolschmelze mit einem kristallisierten Sorbitol in einem Verhältnis kristallisiertes Sorbitol:Sorbitolschmelze oberhalb von 0,5:1, vorzugsweise umfasst von mehr als 1:1 und 2:1,

c) Granulieren des kristallisierten Sorbitols mit der Schmelze bei einer Temperatur des Luftwirbelbetts oberhalb von 65 °C, vorzugsweise umfasst von 75 °C und 85 °C,

d) Gewinnen des granulierten Produkts, welches so erhalten wurde, über das Entladerohr,

e) Abkühlen des granulierten Produkts, welches so erhalten wurde, auf eine Umgebungstemperatur für eine Dauer umfasst von 30 Minuten und 2 Stunden, vorzugsweise gleich 1 Stunde,
f) Sieben und Gewinnen des so erhaltenen granulierten Sorbitols.

7. Verfahren nach Anspruch 6, umfassend den folgenden weiteren Schritt:

g) Recyceln der Stäube, der gemahlenen Rückstände und eines Teils des so erhaltenen granulierten Sorbitols, um ein Verhältnis des Pulvergewichts von Sorbitol:Sorbitolschmelze oberhalb von 0,5:1, vorzugsweise umfasst von 1:1 und 2:1, aufrecht zu erhalten.

8. Verwendung des granulierten Sorbitols nach einem der Ansprüche 1 bis 4 als Süßstoff, Texturmittel, Excipiens und insbesondere als Additivträger in Zusammensetzungen in Tablettenform.

9. Tablette, hergestellt aus granuliertem Sorbitol gemäß einem der Ansprüche 1 bis 4.

## Claims

1. Granulated sorbitol with a content of sorbitol crystals in $\gamma$ form of greater than 95% by weight and a sorbitol content greater than 98% by weight, , having:

   - a specific surface area, determined according to the BET process, greater than or equal to 2 $m^2/g$, preferably comprised between 2.2 and 4 $m^2/g$ and even more preferentially comprised between 2.5 and 3.5 $m^2/g$,
   - a compressibility comprised between 200 and 400 N, preferably comprised between 250 and 350 N, and
   - a volume average diameter measured by LASER diffraction granulometry using a dry-route module comprised between 260 and 1000 $\mu m$, preferably comprised between 260 and 500 $\mu m$, and even more preferentially comprised between 260 and 350 $\mu m$.

2. The Sorbitol according to claim 1, further having a hygroscopicity value determined by a change in weight of the sorbitol between 60% and 0% relative humidity, comprised between 1 and 2%.

3. The Sorbitol according to either one of claims 1 and 2, further having a density comprised between 350 and 650 g/l, preferably comprised between 400 and 550 g/l.

4. The Sorbitol according to any one of claims 1 to 3, further having a free-flow comprised between 5 and 20 seconds, preferably between 5 and 15 seconds.

5. A process for the preparation of the granulated sorbitol according to any one of claims 1 to 4, comprising a step of spraying a sorbitol melt onto a crystallized sorbitol in a fluidized air bed granulator.

6. The Process according to claim 5, consisting of the following series of steps:

   a) optionally, concentrating by evaporation, a solution having a sorbitol dry matter content greater than 65%, preferably comprised between 65 and 75%, more preferentially 70%, in order to obtain a solution having a high dry matter content or a melt,
   b) introducing into a fluidized air bed granulator having a discharge pipe, said sorbitol melt or said sorbitol solution with a high dry matter content with a crystallized sorbitol in a ratio by weight of crystallized sorbitol : sorbitol melt or sorbitol solution with a high dry matter content greater than 0.5 : 1, preferably comprised between 1 : 1 and 2 : 1,
   c) granulating the crystallized sorbitol with said melt, at a fluidized air bed temperature greater than 65°C, preferably comprised between 75°C and 85°C,
   d) recovering the granulated product thus obtained via the discharge pipe,
   e) cooling said granulated product thus obtained at ambient temperature, for a duration comprised between 30 minutes and 2 hours, preferably equal to 1 hour,
   f) sieving and recovering the granulated sorbitol thus obtained.

7. The process according to claim 6 comprising the following subsequent step:

   g) recycling the fines, crushed rejects and a fraction of the granulated sorbitol thus obtained in order to maintain a ratio by weight of sorbitol powder: sorbitol melt or sorbitol solution with a high dry matter content greater than 0.5 : 1, preferably comprising between 1 : 1 and 2 : 1.

8. Use of granulated sorbitol according to any one of claims 1 to 4 as a sweetener, texturizer, excipient or as an additive medium in compositions in tablet form.

9. Tablet manufactured from granulated sorbitol according to any one of claims 1 to 4.

## RÉFÉRENCES CITÉES DANS LA DESCRIPTION

**Documents brevets cités dans la description**

- FR 1506334
- FR 2622190
- EP 32288 A
- EP 380219 A
- GB 20466743 A
- EP 1008602 A

**Littérature non-brevet citée dans la description**

- **S. BRUNAUER et al.** *Journal of American Chemical Society,* 1938, vol. 60, 309